(19) Europäisches Patentamt
European Patent Office
Office européen des brevets

(11) Veröffentlichungsnummer: **0 477 829 B1**

# (12) EUROPÄISCHE PATENTSCHRIFT

(45) Veröffentlichungstag der Patentschrift: **28.06.95**

(21) Anmeldenummer: **91116166.9**

(22) Anmeldetag: **23.09.91**

(51) Int. Cl.6: **C12N 1/20**, C12P 17/12, //(C12N1/20,C12R1:01,1:06)

## (54) Mikrobiologisches Verfahren zur Herstellung von hydroxylierten Heterocyclen.

(30) Priorität: **25.09.90 CH 3091/90**

(43) Veröffentlichungstag der Anmeldung:
**01.04.92 Patentblatt 92/14**

(45) Bekanntmachung des Hinweises auf die Patenterteilung:
**28.06.95 Patentblatt 95/26**

(84) Benannte Vertragsstaaten:
**AT BE CH DE DK ES FR GB IT LI NL SE**

(56) Entgegenhaltungen:
**EP-A- 0 111 160**
**DE-A- 3 903 759**
**US-A- 3 720 768**
**US-A- 4 181 724**

(73) Patentinhaber: **LONZA AG**
**(Geschäftsleitung: 4002 Basel)**
**CH-3945 Gampel/Wallis (CH)**

(72) Erfinder: **Kiener, Andreas, Dr.**
**Meisenweg 5**
**Visp (Kanton Wallis) (CH)**
Erfinder: **van Gameren, Yvonne**
**Mauritsstraat 9**
**Ridderkerk (NL)**
Erfinder: **Bokel, Michael, Dr.**
**Bäretstrasse 4**
**Visp (Kanton Wallis) (CH)**

(74) Vertreter: **Weinhold, Peter, Dr. et al**
**Patentanwälte**
**Dr. Weinhold, Dannenberg,**
**Dr. Gudel, Schubert**
**Siegfriedstrasse 8**
**D-80803 München (DE)**

EP 0 477 829 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Mikroorganismen, die mit 2,5-Dimethylpyrazin wachsen und Pyrazine oder Chinoxaline der allgemeinen Formel

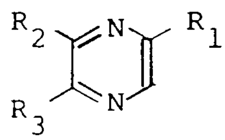

I

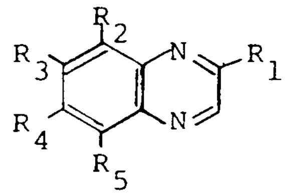

II

hydroxylieren, sowie ein Verfahren zur Herstellung von hydroxylierten Pyrazinen oder Chinoxalinen.

Hydroxylierte Pyrazine sind beispielsweise wichtige Zwischenprodukte zur Herstellung von Methoxyalkylpyrazinen. Methoxyalkylpyrazine sind wesentliche Bestandteile von Aromastoffen (Maga und Sizer, J.Agric.Food Chem., 21, 1973, S.22-30). Hydroxylierte Chinoxaline sind beispielsweise wichtige pharmazeutische Zwischenprodukte (US-PS 4 814 444).

Bisher sind nur chemische Verfahren zur Herstellung von hydroxylierten Chinoxalinen und hydroxylierten Pyrazinen bekannt. Beispielsweise beschreibt die US-PS 4 814 444 ein Verfahren, in welchem 6-Chlor-2-hydroxychinoxalin-4-oxid in Gegenwart eines Katalysators zu 6-Chlor-2-hydroxychinoxalin reduziert wird. Dieses Verfahren hat jedoch den Nachteil, dass es grosstechnisch nicht gangbar ist.
Ein chemisches Verfahren zur Herstellung von hydroxylierten Pyrazinen wird beispielsweise von Karmas und Spoerri in J.Amer.Chem., 74, 1952, S.1580-1584 beschrieben, bei welchem beispielsweise 2-Hydroxy-5-methylpyrazin, ausgehend von Methylglyoxal und Glycinamidhydrochlorid synthetisiert wird. Dieses Verfahren hat jedoch den Nachteil, dass das Produkt stark verunreinigt ist.

Des weiteren sind Untersuchungen über den biologischen Abbau von 2-Hydroxypyrazin in Matley und Harle, Biochem.Soc.Trans., 4, 1976, S.492-493, und Untersuchungen zu 2-Pyrazincarboxamid in Soini und Pakarinen, FEMS Microbiol.Lett., 1985, S.167-171 beschrieben. Es sind jedoch keine Mikroorganismen bekannt, die Pyrazine oder Chinoxaline der allgemeinen Formel I oder II hydroxylieren und dieses im Wachstumsmedium akkumulieren.

Der Erfindung liegt die Aufgabe zugrunde, einen neuen Typ von Mikroorganismus zu finden, der befähigt ist, auf einfache Weise substituierte Pyrazine oder Chinoxaline der allgemeinen Formel I oder II regiospezifisch zu hydroxylieren, sowie ein Verfahren zur Herstellung von hydroxylierten Pyrazinen und Chinoxalinen zu entwickeln.

Diese Aufgabe wurde gemäss Patentanspruch 1 gelöst. Diese Mikroorganismen sind befähigt mit 2,5-Dimethylpyrazin als einzige Kohlenstoff-, Stickstoff- und Energiequelle zu wachsen, und als Substrat Pyrazine der allgemeinen Formel I

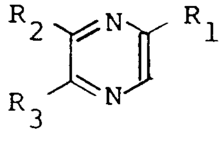

I

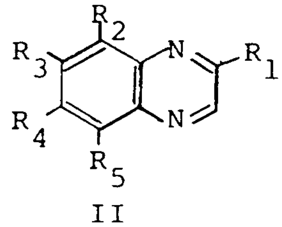

II

oder Chinoxaline der allgemeinen Formel II, worin $R_1$ eine $C_1$-$C_4$-Alkylgruppe oder ein Halogenatom bedeutet und $R_2$, $R_3$, $R_4$ und $R_5$ gleich oder verschieden sind und ein Wasserstoffatom, eine $C_1$-$C_4$-Alkylgruppe oder ein Halogenatom bedeuten, in ein hydroxyliertes Pyrazin der allgemeinen Formel III

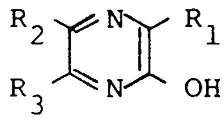

III

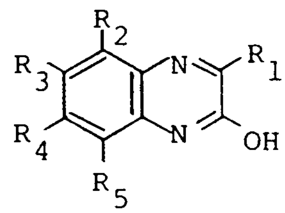

IV

oder in ein hydroxyliertes Chinoxalin der allgemeinen Formel IV, worin $R_1$, $R_2$, $R_3$, $R_4$ und $R_5$ die oben genannte Bedeutung haben, umzusetzen, wobei letztere im Wachstumsmedium akkumuliert werden.

Wie Untersuchungen mit Bodenproben aus Kläranlagen, Erdreich, Ameisenhaufen und Komposthaufen zeigten, sind Mikroorganismen, die 2,5-Dimethylpyrazin abbauen, befähigt, Pyrazine oder Chinoxaline der allgemeinen Formel I oder II zu hydroxylieren. Erfindungsgemäss kommen alle diese Stämme für diese Hydroxylierung in Frage, die 2,5-Dimethylpyrazin als einzige Kohlenstoff-, Stickstoff- und Energiequelle verwerten und nach üblichen mikrobiologischen Techniken selektioniert werden.

Zweckmässig können alle gram-positiven und gram-negativen Mikroorganismen angewendet werden, die 2,5-Dimethylpyrazin abbauen und die Heterocyclen der allgemeinem Formel I oder II als Substrat in Heterocyclen der allgemeinen Formel III oder IV hydroxylieren und im Wachstumsmedium akkumuliert werden.

Bevorzugte Mikroorganismen sind Rhodococcus erythropolis mit der DSM-Nr.6138 und Arthrobacter sp. mit der DSM-Nr.6137.

Da die genaue Identifizierung der Mikroorganismen mit der DSM-Nr.6138 und DSM-Nr.6137 erst nach dem Prioritätsdatum dieser Anmeldung erfolgte, wird im folgenden der Mikroorganismus mit der früheren Bezeichnung Rhodococcus equi Heida (DSM-Nr.6138) nun als Rhodococcus erythropolis (DSM-Nr.6138) und der Mikroorganismus mit der früheren Bezeichnung Micrococcus sp YVG (DSM-Nr.6137) nun als Arthrobacter sp. (DSM-Nr.6137) bezeichnet.

Diese Stämme wurden am 7.9.1990 bei der Deutschen Sammlung von Mikroorganismen (DSM) und Zellkulturen GmbH, Mascherodeweg 1b, 3300 Braunschweig/BRD, hinterlegt.

Wissenschaftliche Beschreibung von Arthrobacter sp. (DSM-Nr.6137)

Charakterisierung: In jungen Kulturen pleomorphe Stäbchen, in älteren Kulturen kokkoid bis Kokken; Gram-positiv; strikt aerob, keine Säurebildung aus Glucose

| | |
|---|---|
| Beweglichkeit | - |
| Sporen | - |
| Katalase | + |

meso-Diaminopimelinsäure in der Zellwand: nein

Peptidoglycan-Typ: A3$\alpha$, Lys-Ala$_{2-3}$; die $\alpha$-Carboxylgruppe der D-Glutaminsäure der Peptiduntereinheit ist durch einen Glycin-Rest substituiert

Wissenschaftliche Beschreibung von Rhodococcus erythropolis (DSM-Nr.6138)

Aminosäure des Peptidoglycans:

| | |
|---|---|
| Diaminopimelinsäure (DAP) | + |

Zucker aus Ganzzellhydrolysaten:

| | |
|---|---|
| Arabinose (ARA) | + |
| Galactose (GAL) | + |
| Madurose (MAD) | - |
| Xylose (XYL) | - |
| Glukose (GLU) | + |
| Ribose (RIB) | + |

Fettsäuren: unverzweigte gesättigte und ungesättigte Fettsäuren plus Tuberculostearinsäure vorhanden 15-30%

Mycolsäuren: Mycolsäuren mit einer Kettenlänge von $C_{35}$-$C_{40}$ vorhanden

Menachinone: Typ MK-8 ($H_2$) 93%

Physiologische Tests zur Spezies-Identifizierung mycolsäurehaltiger Mikroorganismen:

| | |
|---|---|
| N-Acetylglucosamin (NAG) | + |
| D-Glucosaminsäure (GAT) | - |
| D-Turanose (TUR) | - |
| 2-Hydroxyvalerat (o2V) | + |
| L-Alanin (ALA) | + |
| L-Prolin (PRO) | - |
| Tyramin (TYR) | - |
| 4-Aminobutyrat (o4B) | + |
| 2-Desoxythymidin-s-pup-phosphat (CDP) | + |
| Galactose (GAL) | - |
| L-Rhamnose (RHA) | - |
| Aralit (ARA) | + |
| 2-Oxo-glutarat (o2G) | - |
| 4-Aminobutyrat (a4B) | - |
| L-Serin (SER) | + |
| Acetamid (ATA) | + |
| Quinat (QUI) | + |
| D-Glucarat (GCT) | - |
| D-Ribose (RiB) | + |
| Inosit (INO) | + |
| Pimelas (PIM) | + |
| L-Aspartat (ASP) | - |
| L-Valin (VAL) | + |
| Benzoat (BEN) | - |
| pNP-beta-D-xylosid (CXY) | + |
| Gluconat (GDT) | + |
| Sucrose (D-Saccharose; SUC) | + |
| Citrat (CIT) | + |
| Succinat (SAT) | + |
| L-Leucin (LEU) | + |
| Putrescin (PUT) | + |
| 3-Hydroxybenzoat (o3B) | - |
| pNP-phosphoryl-cholin (CCH) | + |

Farb-Code für Kolonien coryneformer Organismen: Typ Nr.60 nach Seiler (1983)

Für das Verfahren zur Herstellung von hydroxylierten Pyrazinen oder Chinoxalinen werden mit den Mikroorganismen ein Pyrazin der allgemeinen Formel I

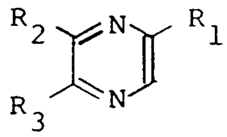

I

oder ein Chinoxalin der allgemeinen Formel II

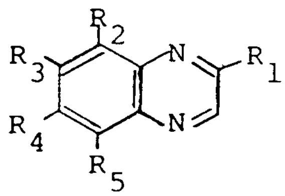

II

worin $R_1$ eine $C_1$-$C_4$-Alkylgruppe oder ein Halogenatom bedeutet und $R_2$, $R_3$, $R_4$ und $R_5$ gleich oder verschieden sind und ein Wasserstoffatom, eine $C_1$-$C_4$-Alkylgruppe oder ein Halogenatom bedeutet, in ein hydroxyliertes Pyrazin der allgemeinen Formel III

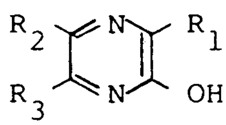

III

oder in ein hydroxyliertes Chinoxalin der allgemeinen Formel IV,

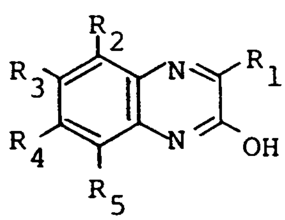

IV

worin $R_1$, $R_2$, $R_3$, $R_4$ und $R_5$ die oben genannte Bedeutung haben, überführt und das angereicherte Produkt isoliert.

Als zu hydroxylierende Pyrazine oder Chinoxaline kommen Verbindungen der allgemeinen Formel I oder II, in Frage.

Vorzugsweise werden durch diese Mikroorganismen Pyrazinderivate oder Chinoxalinderivate hydroxyliert worin $R_1$ eine Methyl-, Ethylgruppe oder ein Chloratom und $R_2$ und $R_3$ gleich oder verschieden sind und ein Wasserstoffatom, eine Methylgruppe oder ein Chloratom bedeuten.

Ueblicherweise werden die Mikroorganismen vor dem eigentlichen Verfahren (Substratumsetzung) in einem Medium, enthaltend ein Wachstumssubstrat, kultiviert.

Das Wachstumssubstrat 2,5-Dimethylpyrazin wird zweckmässig in einer Menge von 0,001 bis 10% (w/v), bezogen auf das Kulturmedium, vorzugsweise in einer Menge von 0,001 bis 5% (w/v), bezogen auf das Kulturmedium, angewendet.

Die zur Hydroxylierung verantwortlichen Enzyme des Mikroorganismus werden zweckmässig durch 2,5-Dimethylpyrazin induziert.

Die zur Induktion verwendete Verbindung kann entweder während der Umsetzung des Heterocyclussubstrats anwesend sein oder die Zufuhr dieser Verbindung wird während der Umsetzung unterbunden.
Vorzugsweise wird die Zufuhr der zur Induktion verwendeten Verbindung während der Umsetzung des

Heterocyclussubstrats entweder durch Stoppen der Zufuhr oder durch beispielsweise Zentrifugieren der Zellen unterbunden.

Vor der Substratzugabe werden die Zellen zweckmässig bis zu einer optischen Dichte von 100 bei 650 nm, vorzugsweise bis zu einer optischen Dichte von 10 bis 60 bei 650 nm angezogen.

Als Nährmedium für die Mikroorganismen können sowohl für die Kultivierung als auch für das eigentliche Verfahren die in der Fachwelt üblichen angewendet werden, vorzugsweise wird das Medium, dessen Zusammensetzung in Tabelle 1 angegeben ist, angewendet.

Das eigentliche Verfahren (Substratumsetzung) erfolgt dann üblicherweise mit ruhenden Zellen.

Das Pyrazin oder Chinoxalin der allgemeinen Formel I oder II kann einmalig oder kontinuierlich zur Zellsuspension zugeführt werden, vorzugsweise so, dass die Substrat-Konzentration im Kulturmedium 20% (w/v) nicht übersteigt. Insbesondere übersteigt die Substrat-Konzentration nicht 5% (w/v) im Kulturmedium.

Die Umsetzung wird in einem pH-Bereich von 4 bis 10 , vorzugsweise von 6 bis 8 durchgeführt.

Ueblicherweise wird die Umsetzung bei einer Temperatur von 0 bis 50 °C, vorzugsweise bei 20 bis 40 °C, durchgeführt.

Nach der Umsetzung können die hydroxylierten Heterocyclen auf bekannte Weise, z.B. durch Extraktion mit chlorierten Kohlenwasserstoffen, isoliert werden.

Beispiel 1

Isolierung von 2,5-Dimethylpyrazin-metabolisierenden Mikroorganismen

Aerobe 2,5-Dimethylpyrazin-metabolisierende Mikroorganismen wurden im A + N-Medium (Tabelle 1) mit Zusatz von 0,1% (w/v) 2,5-Dimethylpyrazin als einzige Kohlenstoff-, Stickstoff- und Energiequelle angereichert. Die allgemeinen Techniken zur Isolation von Mikroorganismen sind beispielsweise in "G.Drews, Mikrobiologisches Praktikum, 4 Aufl., Springer Verlag, 1983" beschrieben.
Als Inokulum wurden Proben aus dem Erdreich, Kläranlagen, Kompost und Ameisenhaufen verwendet. Die Anreicherungen wurden in Schüttelkolben bei 30 °C angezogen. Nach dreimaligem Ueberimpfen in frisches Medium wurden die Anreicherungen auf dem gleichen Medium mit Zusatz von 16 g Agar pro Liter ausgestrichen und bei 30 °C inkubiert. Nach mehrmaligem Ausstreichen auf Agarmedium konnten Reinkulturen isoliert werden.

Tabelle 1: A+N-Medium

| Zusammensetzung: | Konzentration (mg/l) |
|---|---|
| $(NH_4)_2SO_4$ | 2000 |
| $Na_2HPO_4$ | 2000 |

| | |
|---|---|
| $KH_2PO_4$ | 1000 |
| NaCl | 3000 |
| $MgCl_26 \cdot H_2O$ | 400 |
| $CaCl_2 \cdot 2H_2O$ | 14,5 |
| $FeCl_3 \cdot 6H_2O$ | 0,8 |
| Pyridoxal-Hydrochlorid | $10 \cdot 10^{-3}$ |
| Riboflavin | $5 \cdot 10^{-3}$ |
| Nicotinsäureamid | $5 \cdot 10^{-3}$ |
| Thiamin-Hydrochlorid | $2 \cdot 10^{-3}$ |
| Biotin | $2 \cdot 10^{-3}$ |
| Panthothensäure | $5 \cdot 10^{-3}$ |
| p-Aminobenzoat | $5 \cdot 10^{-3}$ |
| Folsäure | $2 \cdot 10^{-3}$ |
| Vitamin B12 | $5 \cdot 10^{-3}$ |
| $ZnSO_4 \cdot 7H_2O$ | $100 \cdot 10^{-3}$ |
| $MnCl_2 \cdot 4H_2O$ | $90 \cdot 10^{-3}$ |
| $H_3BO_3$ | $300 \cdot 10^{-3}$ |
| $CoCl_2 \cdot 6H_2O$ | $200 \cdot 10^{-3}$ |
| $CuCl_2 \cdot 2H_2O$ | $10 \cdot 10^{-3}$ |
| $NiCl_2 \cdot 6H_2O$ | $20 \cdot 10^{-3}$ |
| $Na_2MoO_4 \cdot 2H_2O$ | $30 \cdot 10^{-3}$ |
| $EDTANa_2 \cdot 2H_2O$ | $5 \cdot 10^{-3}$ |
| $FeSO_4 \cdot 7H_2O$ | $2 \cdot 10^{-3}$ |

(pH der Lösung wurde auf 7.0 eingestellt)

## Beispiel 2

### Umsetzung von 2,5-Dimethylpyrazin zu 2,5-Dimethyl-3-hydroxypyrazin

Rhodococcus erythropolis (DSM-Nr.6138) wurde im A+N-Medium mit 0,1% (w/v) 2,5-Dimethylpyrazin in einem Fermenter bei pH 7 und einer Temperatur von 25°C angezogen. Anschliessend wurden die Zellen zentrifugiert und im A+N-Medium resuspendiert und eine optische Dichte bei 650 nm von 10 eingestellt. Diese Zellsuspension wurde in einen Schüttelkolben gegeben und mit 92 mmol 2,5-Dimethylpyrazin pro Liter versetzt. Nach einer Inkubation bei 25°C von 4 h auf einer Schüttelmaschine wurden 83 mmol 2,5-Dimethyl-3-hydroxypyrazin pro Liter, entsprechend einer Ausbeute von 90%, nachgewiesen.

Die Beispiele 3 bis 9 wurden entsprechend zu Beispiel 2 durchgeführt und sind in Tabelle 2 zusammengefasst.

Tabelle 2

| Beispiel | Substrat | Konz.des Heterocyclus in % (w/v) im Medium | Reakt. zeit in h | Endprodukt | Ausbeute in % |
|---|---|---|---|---|---|
| 3 | 2-Methylpyrazin | 0,2 | 1 | 3-Hydroxy-2-methylpyrazin | 90 |
| 4 | 2-Chlorpyrazin | 0,2 | 24 | 3-Hydroxy-2-chlorpyrazin | 60 |
| 5 | 2-Ethylpyrazin | 0,2 | 24 | 3-Hydroxy-2-ethylpyrazin | 80 |
| 6 | 2,6-Dimethylpyrazin | 0,2 | 1 | 3-Hydroxy-2,6-dimethylpyrazin | 90 |
| 7 | 2,5,6-Trimethylpyrazin | 0,2 | 6 | 3-Hydroxy-2,5,6-trimethylpyrazin | 90 |
| 8 | 6-Chlor-2,5-dimethylpyrazin | 0,2 | 1 | 3-Hydroxy-6-chlor-2,5-dimethylpyrazin | 90 |
| 9 | 2-Methylchinoxalin | 0,4 | 5 | 3-Hydroxy-2-methylchinoxalin | 50 |

## Patentansprüche

**1.** Mikroorganismen, dadurch gekennzeichnet, daß sie befähigt sind, mit 2,5-Dimethylpyrazin als einziger Kohlenstoff-, Stickstoff- und Energiequelle zu wachsen und als Substrat Pyrazine der allgemeinen Formel I oder Chinoxaline der allgemeinen Formel II

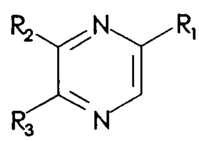

I

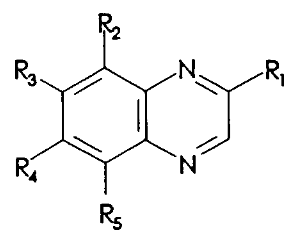

II

worin $R_1$ eine $C_1$-$C_4$-Alkylgruppe oder ein Halogenatom bedeutet und $R_2$, $R_3$, $R_4$ und $R_5$ gleich oder verschieden sind und ein Wasserstoffatom, eine $C_1$-$C_4$-Alkylgruppe oder ein Halogenatom bedeuten, unter Akkumulierung der Endprodukte im Wachstumsmedium in hydroxylierte Pyrazine der allgemeinen Formel III oder hydroxylierte Chinoxaline der allgemeinen Formel IV

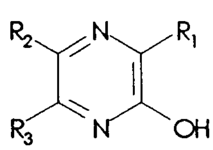

III

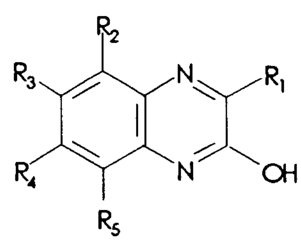

IV

worin $R_1$, $R_2$, $R_3$, $R_4$ und $R_5$ die oben genannte Bedeutung haben, umzusetzen.

**2.** Mikroorganismus nach Patentanspruch 1 mit der Bezeichnung Rhodococcus erythropolis, hinterlegt bei der DSM mit der Nummer 6138, und dessen Deszendenten und Mutanten.

3. Mikroorganismus nach Patentanspruch 1 mit der Bezeichnung Arthrobacter sp., hinterlegt bei der DSM mit der Nummer 6137, und dessen Deszendenten und Mutanten.

4. Verfahren zur Herstellung von hydroxylierten Pyrazinen und Chinoxalinen, dadurch gekennzeichnet, dass man mit den Mikroorganismen gemäss einem der Patentansprüche 1, 2 oder 3 ein Pyrazin der allgemeinen Formel

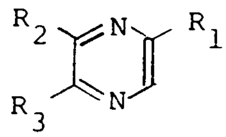

I

oder ein Chinoxalin der allgemeinen Formel

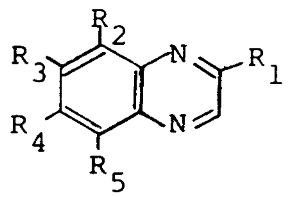

II

worin $R_1$ eine $C_1$-$C_4$-Alkylgruppe oder ein Halogenatom bedeutet und $R_2$, $R_3$, $R_4$ und $R_5$ gleich oder verschieden sind und ein Wasserstoffatom, eine $C_1$-$C_4$-Alkylgruppe oder ein Halogenatom bedeutet, in ein hydroxyliertes Pyrazin der allgemeinen Formel III

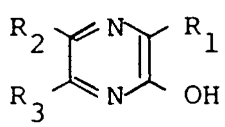

III

oder in ein hydroxyliertes Chinoxalin der allgemeinen Formel IV,

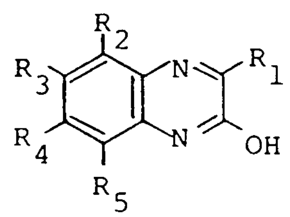

IV

worin $R_1$, $R_2$, $R_3$, $R_4$ und $R_5$ die oben genannte Bedeutung haben, überführt und das angereicherte Produkt isoliert.

5. Verfahren nach Patentanspruch 4, dadurch gekennzeichnet, dass die wirksamen Enzyme des Mikroorganismus mit 2,5-Dimethylpyrazin induziert werden.

6. Verfahren nach mindestens einem der Patentansprüche 4 oder 5, dadurch gekennzeichnet, dass die Umsetzung unter einmaliger oder kontinuierlicher Substrat-Zugabe durchgeführt wird, so dass die Substrat-Konzentration im Kulturmedium 20% (w/v) nicht übersteigt.

7. Verfahren nach mindestens einem der Patentansprüche 4 bis 6, dadurch gekennzeichnet, dass die Umsetzung bei einem pH von 4 bis 10 durchgeführt wird.

8. Verfahren nach mindestens einem der Patentansprüche 4 bis 7, dadurch gekennzeichnet, dass die Umsetzung bei Temperaturen von 0 bis 55 ° C durchgeführt wird.

## Claims

1. Microorganisms, characterized in that they are capable of growing on 2,5-dimethylpyrazine as the sole source of carbon, nitrogen and energy and of transforming, as substrate, pyrazines of the general formula I or quinoxalines of the general formula II

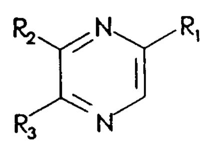

I

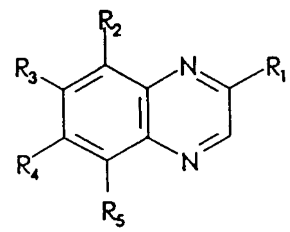

II

wherein $R_1$ represents a $C_1$-$C_4$-alkyl group or a halogen atom and $R_2$, $R_3$, $R_4$ and $R_5$ are the same or different and represent a hydrogen atom, a $C_1$-$C_4$-alkyl group or a halogen atom, by accumulating the end products in the growth medium to hydroxylated pyrazines of the general formula III or hydroxylated quinoxalines of the general formula IV

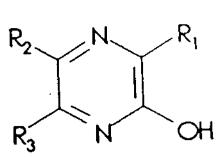

III

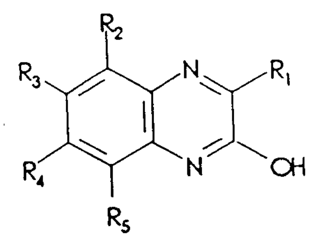

IV

wherein $R_1$, $R_2$, $R_3$, $R_4$ and $R_5$ are as defined above.

2. Microorganism according to claim 1 with the designation Rhodococcus erythropolis, deposited at the DSM under the number 6138, and descendants and mutants thereof.

3. Microorganism according to claim 1 with the designation Arthrobacter sp., deposited at the DSM under the number 6137, and descendants and mutants thereof.

4. Process for preparing hydroxylated pyrazines and quinoxalines, characterized in that a pyrazine of the general formula I

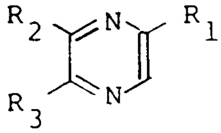

I

or a quinoxaline of the general formula II

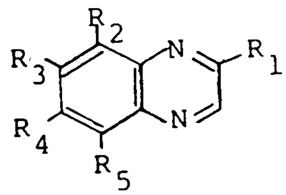

II

wherein $R_1$ represents a $C_1$-$C_4$-alkyl group or a halogen atom and $R_2$, $R_3$, $R_4$ and $R_5$ are the same or different and represent a hydrogen atom, a $C_1$-$C_4$-alkyl group or a halogen atom, is transformed with the microorganisms according to any one of claims 1, 2 or 3 to a hydroxylated pyrazine of the general formula III

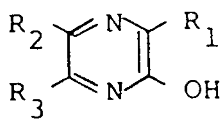

III

or to a hydroxylated quinoxaline of the general formula IV

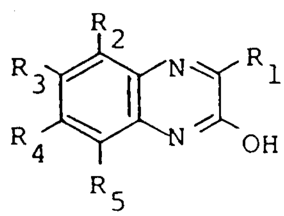

IV

wherein $R_1$, $R_2$, $R_3$, $R_4$ and $R_5$ are as defined above, and that the enriched product is isolated.

5. Process according to claim 4, characterized in that the effective enzymes of the microorganism are induced with 2,5-dimethylpyrazine.

6. Process according to at least one of claims 4 or 5, characterized in that the transformation is carried out by a single or a continuous substrate addition in a way that the substrate concentration in the culture medium does not exceed 20% (w/v).

7. Process according to at least one of claims 4 to 6, characterized in that the transformation is carried out at a pH of from 4 to 10.

8. Process according to at least one of claims 4 to 7, characterized in that the transformation is carried out at temperatures of from 0 to 55 ° C.

**Revendications**

1. Microorganismes, caractérisés en ce qu'ils sont aptes à pousser, en présence de la 2,5-diméthylpyrazine comme source unique de carbone, d'azote et d'énergie et, en tant que substrat, à transformer les pyrazines de la formule générale I ou les quinoxalines de la formule générale II,

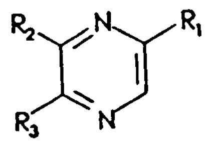

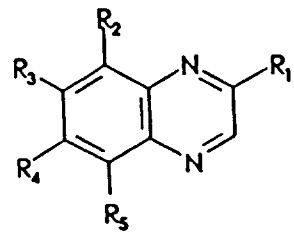

I II

formule dans laquelle $R_1$ signifie un groupe alkyle $C_1$-$C_4$ ou un atome halogène et $R_2$, $R_3$, $R_4$ et $R_5$ sont identiques ou différents et signifient un atome hydrogène, un groupe alkyle $C_1$-$C_4$ ou un atome halogène, par accumulation des produits finaux dans le milieu de culture en pyrazines hydroxylées de la formule générale III ou en quinoxalines hydroxylées de la formule générale IV,

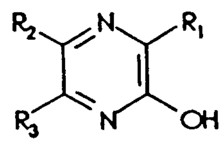

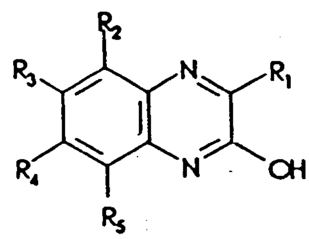

III IV .

dans laquelle $R_1$, $R_2$, $R_3$, $R_4$ et $R_5$ ont la signification précitée.

2. Microorganisme selon la revendication 1, portant la désignation rhodococcus erythropolis, déposé auprès de DSM sous le numéro 6138 ainsi que ses descendants et mutants.

3. Microorganisme selon la revendication 1, portant la désignation arthrobacter sp., déposé auprès de DSM sous le numéro 6137 ainsi que ses descendants et mutants.

4. Procédé pour la préparation de pyrazines hydroxylées et de quinoxalines, caractérisé en ce qu'avec les microorganismes selon l'une des revendications 1, 2 ou 3 l'on transforme une pyrazine de la formule générale

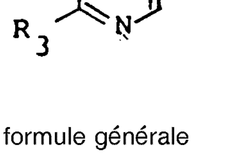

I

ou une quinoxaline de la formule générale

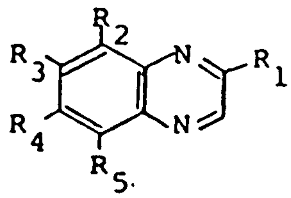

II

dans laquelle $R_1$ signifie un groupe alkyle $C_1$-$C_4$ ou un atome halogène, et $R_2$, $R_3$, $R_4$ et $R_5$ sont identiques ou différents et un atome d'hydrogène, un groupe alkyle $C_1$-$C_4$ ou un atome d'halogène en une pyrazine hydroxylée de la formule générale III

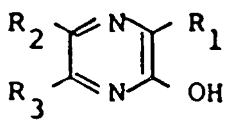

III

ou en une quinoxaline hydroxylée de la formule générale IV,

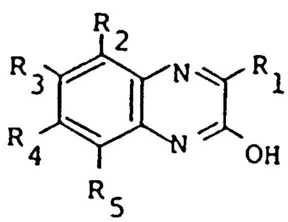

IV

dans laquelle $R_1$, $R_2$, $R_3$, $R_4$ et $R_5$ ont la signification précitée et on isole le produit enrichi.

**5.** Procédé selon la revendication 4, caractérisé en ce que les enzymes efficaces du microorganisme sont induites avec la 2,5-diméthylpyrazine.

**6.** Procédé selon au moins l'une des revendications 4 ou 5, caractérisé en ce la transformation ou réaction est effectuée par addition de substrat en une seule fois de façon continue de sorte que la concentration de substrat dans le milieu de culture ne dépasse pas 20% (p/v).

**7.** Procédé selon au moins l'une des revendications 4 à 6, caractérisé en ce que la réaction s'effectue à un pH de 4 à 10.

**8.** Procédé selon au moins l'une des revendications 4 à 7, caractérisé en ce que la réaction s'effectue à des températures de 0 à 55 °C.